# EUROPÄISCHE PATENTSCHRIFT

(11) **EP 1 857 802 B1**
(45) Veröffentlichungstag und Bekanntmachung des Hinweises auf die Patenterteilung: **27.02.2013**
(21) Anmeldenummer: 07009603.7
(22) Anmeldetag: 14.05.2007
(51) Int. Cl.: G01N 15/02, G01N 33/18, G21C 17/022

(54) **Verfahren zur automatischen Überwachung der Qualität der Wasser-Dampf-Kreisläufe in Kraftwerken**
Automatic quality control method for the water-vapour cycles in power stations
Procédé destiné à la surveillance automatique de la qualité des circuits eau-vapeur dans des centrales

(30) Priorität: 20.05.2006 DE 102006023793
(43) Veröffentlichungstag der Anmeldung: 21.11.2007
(73) Patentinhaber: Jenderek, Henry, 72813 St. Johann (DE)
(72) Erfinder: Rudorf, Jürgen, 50769 Köln (DE)
(74) Vertreter: Ruckh, Rainer Gerhard

(56) Entgegenhaltungen:
- EP-A- 1 102 054
- US-A- 5 708 198
- US-A- 5 918 272
- US-A1- 2005 043 901

## Beschreibung

Derzeit werden Monitoring-, Analyse- und Diagnosesysteme für Kraftwerke gesucht, um durch ständige Diagnose und in Verbindung mit dem Wissen über die ablaufenden Prozesse Management-Systeme zur Optimierung der Leistung und Verfügbarkeit zu entwickeln. Durch die Kombination unterschiedlichster Messdaten sollen Expertensysteme in die Lage versetzt werden, den Anlagenzustand weitgehend selbsttätig und ohne die Erfordernis, zusätzliche Laboruntersuchungen durchzuführen, zu beurteilen und dem Anlagenbetreiber Handlungsempfehlungen zu geben.

Zur Überwachung der Qualität des Wasser-Dampf-Kreislaufes werden heute sowohl Online-Messungen wie auch Laboranalyse-Verfahren kombiniert, um die als relevant erkannten Parameter mindestens quasi-kontinuierlich zu überwachen. Hier seien insbesondere die automatisierte Messung der Leitfähigkeit und des pH-Wertes sowie die in laboranalytischen Verfahren vorgenommene diskontinuierliche Eisen-Bestimmung erwähnt.

Die zu überwachenden Analysenparameter und die an den einzelnen Stellen im Wasser-Dampf-Kreislauf einzuhaltenden Richtwerte sind in nationalen und internationalen Normen festgelegt, z.B. in der VGB-Richtlinie R 450 Le "Guidelines for Feed Water, Boiler Water and Steam Quality for Power Plants/Industrial Plants - 2./2004". Dabei werden insbesondere an die Reinheit des Heißdampfes vor der Dampfturbine hohe Anforderungen gestellt.

Die festgelegten Richtwerte können erfahrungsgemäß nicht unter allen Betriebsbedingungen, insbesondere nicht sofort beim Anfahrbetrieb des Kraftwerkes, eingehalten werden. Aus diesem Grunde wurden so genannte "Action Levels" definiert, die beschreiben, über welchen Zeitraum erhöhte Konzentrationswerte für die elektrische Leitfähigkeit bzw. erhöhte Konzentrationen an Eisen und Kieselsäure im Heißdampf vor der Dampfturbine noch zulässig sind.

Die Einhaltung solcher Richtwerte ist oft Gegenstand von Garantievereinbarungen. Zum Schutz der Dampfturbine darf diese nach den anerkannten Regeln der Technik erst dann mit Heißdampf beaufschlagt werden, wenn wenigstens diese erhöhten Konzentrationen unterschritten sind. Bis zu diesem Zeitpunkt wird der Dampf zum Beispiel entweder entspannt und mit Kühlwasser kondensiert als Speisewasser im Kreislauf oder aber direkt über Dach gefahren, was mit hohen Energieverlusten verbunden ist. Zugleich führt dies zu einer Belastung der Umwelt, da die zur Erzeugung des Dampfes eingesetzte Energie aus fossilen oder anderen Quellen irreversibel verbraucht und dann ausschließlich zur Aufheizung der Atmosphäre vergeudet wurde.

In der Praxis scheiterte die vollautomatische Qualitätsüberwachung des Wasser-Dampf-Kreislaufes während dieser Anfahrphasen wie auch im laufenden Betrieb der Kraftwerke bisher insbesondere daran, dass keine zuverlässigen, wartungsarmen und kostengünstigen Online-Messgeräte zur Bestimmung der ungelösten Eisenverbindungen zur Verfügung stehen. Dies ist ganz offensichtlich, da selbst in modernen Kraftwerksneubauten solche Messeinrichtungen nicht als Ersatz für die stets vorgeschriebenen Laboranalysen vorgesehen werden.

Ein Standardverfahren zur Bestimmung des Gesamteisens im Kraftwerkslabor basiert auf dem Aufschluss der Eisenverbindungen in der Wasserprobe, z.B. mit Thioglykolsäure über 30 Minuten bei 90°C, der Abkühlung der Probe und der nachfolgenden photometrischen Bestimmung des mit 1,10 - Phenanthrolin gebildeten orangeroten Komplexes, der im pH - Bereich 2,5 - 9 stabil ist und photometrisch nach einer Wartezeit von mindestens 10 Minuten nach Reagenzzugabe bei einer Wellenlänge von 510 nm vermessen wird, oder vergleichbaren Verfahren, wie unter anderem beschrieben im VGB-Handbuch B 401 "Chemie im Kraftwerk" - "Analysenverfahren".

Um dabei Minderbefunde, zum Beispiel resultierend aus der Anlagerung der ungelösten Eisenverbindungen an der Wand der Probenahmegefäße, beispielsweise auch durch nachträgliche Abscheidung ehemals gelöster Bestandteile nach der Probenahme, zu vermeiden, muss der Aufschluss direkt im Probenahmegefäß durchgeführt werden. Der Nachteil dieses und ähnlicher Laborverfahren, insbesondere beim Anfahrbetrieb, ist die lange Zeitdauer bis zum Vorliegen des Analyse-Ergebnisses. Aufgrund des erforderlichen Aufschlusses ist die Automatisierung dieses photometrischen Analysenverfahrens für die Online-Betriebsüberwachung sehr aufwendig und wird daher in der Praxis üblicherweise nicht eingesetzt.

Zur Verkürzung der Analysenzeit wurde schon vor Jahrzehnten die Membranfiltermethode eingeführt. Dabei wird ein größeres, abgemessenes Volumen der Wasserprobe über einen Membranfilter (0.45 µm) abgesaugt, anschließend wird z. B. bei einer als "Schnelltest" allgemein bekannten Variante dieser Methode die Filteroberfläche visuell gegen Farbvergleichstafeln (z.B. der Fa. Satorius, Filter Nr. SM 11306) oder photometrisch ausgewertet. Auch dieser relativ ungenaue "Schnelltest" ist recht zeitaufwendig und erfordert im Anfahrbetrieb den zusätzlichen Einsatz von Personal zur Überwachung der Dampfreinheiten. Obwohl bei diesem Verfahren nur die ungelösten Eisenverbindungen erfasst werden, hat es sich in der Praxis prinzipiell gut bewährt und wird auch heute verbreitet eingesetzt.

Im Bestreben, den Personaleinsatz weiter zu reduzieren, wird in manchen Kraftwerken auf die Überwachung der Konzentration der Eisenverbindungen selbst beim Anfahrbetrieb ganz verzichtet. Stattdessen wird eine gewisse Erfahrungs- oder Schätzzeit abgewartet, bevor der Heißdampf zur Dampfturbine geleitet wird. Diese Verfahrensweise ist aber als riskant anzusehen, insbesondere wenn auf Grund der Wettbewerbssituation auf dem Strommarkt die Häufigkeit der An- und Abfahrvorgänge in den Kraftwerken zunehmen. Als Folge davon treten bei erhöhten Eisengehalten im Dampf Ablagerungen auf den Turbinenschaufeln auf, die sowohl Wirkungsgrad als auch Nutzungsdauer reduzieren und die nur aufwendig wieder entfernt werden können.

In einer jüngeren Forschungsarbeit (H.J. Betten, E.V. Maughan, und Dr. H.D. Pflug: "Online Monitoring of Corrosive Tendencies of Cycle Fluid", VGB PowerTech 9/2004. S. 31-37) wurde versucht den Nachweis zu erbringen, dass die Konzentration der Eisenoxide im Wasser-Dampf-Kreislauf mit einer einfachen Online-Trübungsmessung - nach aufwendigen Voruntersuchungen je Probenahmestelle - ausreichend genau bestimmt werden kann. Die in dieser Arbeit nochmals dargestellte Theorie der Trübungsmessung als auch eigene Messungen zeigen aber, dass gerade im hier interessierenden sehr niedrigen Konzentrationsbereich (Richtweite um 0,005 - 0,020 mg/l), z. B. im Hochdruck-Heißdampf, eine zuverlässige Bestimmung der Eisenkonzentration auf der Grundlage einer Trübungsmessung nicht möglich ist, ebenso nicht, wenn die Beschaffenheit der Eisenverbindungen (Teilchengröße bzw. Größenverteilung, Art der Eisenverbindung) stärker variiert. Gerade das ist bei Abweichungen vom Normalbetrieb aber zu erwarten und kann generell nicht ausgeschlossen werden. Deshalb kann die Trübungsmessung keinesfalls eine zuverlässige Grundlage für die automatische Überwachung der Qualität des Wasser-Dampf-Kreislaufes im Anfahrbetrieb eines Kraftwerkes sein.

Im Bereich der Pharma- und Elektronikindustrie werden zur Beurteilung der Reinstwasserqualität Partikelmessgeräte eingesetzt. Im Gegensatz dazu werden in der Kraftwerkschemie zur Beurteilung der Reinheit im Wasser-Dampf-Kreislauf von konventionellen Kraftwerken keine Richtwerte zur Begrenzung der Anzahl oder Größe von Partikeln definiert und vorgeschrieben, so dass hier auf entsprechende Messungen verzichtet wird. Eine Ausnahme ist dabei die Überwachung des Dampfausblasvorganges vor der Inbetriebnahme neuer Anlagenteile und Rohrleitungen. Hier wird die Partikelfreiheit des Dampfes an Hand der Einschlagspuren auf z. B. einem Spiegel visuell beurteilt.

In der US 5,708,198 A ist ein Verfahren zur Partikelbestimmung in Flüssigkeiten beschrieben. In einem ersten Schritt werden mittels eines Partikelzählers Partikel unterschiedlicher Art in einer Flüssigkeit erfasst. Dann werden Eisen-Partikel aus der Flüssigkeit ausgefiltert, um danach mittels eines Partikelzählers Nicht-Eisen-Partikel zu zählen.

Die US 5,918,272 A betrifft ein Verfahren zur Bestimmung der Größenverteilungen von Partikeln in einer Wasserprobe eines Reaktors. Dabei werden mittels eines Magnetfilters zuerst magnetische Partikel ausgefiltert. Die den Filter durchsetzenden Partikel werden im Partikelzähler erfasst. Durch Variation des Magnetfelds im Magnetfilter kann die Art der im Filter ausgefilterten Partikel variiert werden.

In der US 2005/0043901 A1 ist ein Verfahren beschrieben, mittels dessen in Flüssigkeiten von Maschinen enthaltene Partikel analysiert werden. Dabei werden aus den Messwerten eines Partikelzählers Kenngrößen wie die Partikelgrößenverteilung und das Partikelgesamtvolumen bestimmt.

Die EP 1 102 054 A2 betrifft ein abrasives Partikeldetektionssystem. Aus einem Flüssigkeitsstrom wird ein Teilstrom abgezweigt. Als Probenelement wird ein leitfähiger Film auf ein Substrat aufgebracht und in den Teilstrom eingeführt. Abrasive Partikel im Teilstrom tragen den leitfähigen Film auf. Dadurch ändert sich irreversibel die Leitfähigkeit des Films als relevante Messgröße

Ziel der Erfindung ist es, eine vollautomatische Überwachung der Qualität der Wasser-Dampf-Kreisläufe von Kraftwerken im Anfahr- und Normalbetrieb durch eine mindestens quasikontinuierliche Überwachung des Gehaltes an ungelöstem Eisen an allen Probenahmestellen zuverlässig und kostengünstig zu ermöglichen. Sie dient speziell dem Schutz und der Minimierung der Anfahrzeit von Dampfturbinen, der Optimierung der Trommelkesselwasserabschlämmung sowie allgemein der Online-Überwachung von Korrosionsvorgängen in Wasser- und Wasser-Dampf-Kreisläufen.

Es bestand somit die Aufgabe, die bekannten automatisierten Messverfahren im Kraftwerk durch ein ausreichend zuverlässiges Verfahren zur schnellen Konzentrationsbestimmung ungelöster Eisenverbindungen zu ergänzen, um
a) die Anfahrzeiten der Dampfturbine(n) soweit wie möglich verkürzen zu können ohne dabei vermeidbare Wirkungsgradverluste oder Schäden oder Dampfturbinenreinigungen, verursacht durch verunreinigten Heißdampf, in Kauf nehmen zu müssen und
b) um auch im Dauerbetrieb (Normalbetrieb) jederzeit, auch ohne Laborpersonal, die Trommelabschlämmung in Abhängigkeit von der Verschmutzung des Kesselwassers (mit ungelösten Feststoffen, in der Regel Eisenoxiden) so steuern zu können, dass die Gefahr einer Verunreinigung des Dampfes reduziert wird sowie um
c) die Einsatzzeiten der Kondensatreinigung zu steuern und um
d) Korrosionsvorgänge sicher erkennen, dokumentieren und unmittelbar über ein Prozessleitsystem im Zusammenhang mit den übrigen Betriebsdaten auswerten zu können.

Zur Lösung dieser Aufgabe sind die Merkmale des Anspruchs 1 vorgesehen. Vorteilhafte Ausführungsformen und zweckmäßige Weiterbildungen der Erfindung sind in den Unteransprüchen beschrieben.

Das erfindungsgemäße Verfahren dient zur automatischen Überwachung der Qualität von Wasser-Dampf-Kreisläufen in Kraftwerken und umfasst folgende Verfahrensschritte:
a) Bestimmung von Anzahlen und Größenklassen von Partikeln mittels wenigstens eines Online-Partikelzählers an einer Stelle im Wasser-Dampf-Kreislauf,
b) Bestimmung des funktionellen Zusammenhangs zwischen Partikelgröße und Partikelanzahl und anschließende Überprüfung des Vorliegens eines linearen Zusammenhangs zwischen dem Logarithmus der Partikelgröße und dem Logarithmus der Partikelanzahl,
c) Berechnung des Partikelgesamtvolumens und
d) Extrapolation des ermittelten funktionellen Zusammenhangs zwischen Partikelgröße und Partikelanzahl, um im Wasser-Dampf-Kreislauf vorhandene kleinere Partikel, deren Größen unterhalb der Größenklassen-Erfassungsgrenze des verwendeten Online-Partikelzählers liegen, zu bestimmen, wobei diese bei der Berechnung des Partikelgesamtvolumens mitberücksichtigt werden,
e) Berechnung der Konzentration von ungelöstem Eisen aus dem Partikelgesamtvolumen über einen Kalibrier- oder Näherungsfaktor, der sich aus der Dichte und dem Eisengehalt der ungelösten Eisenverbindungen ergibt.

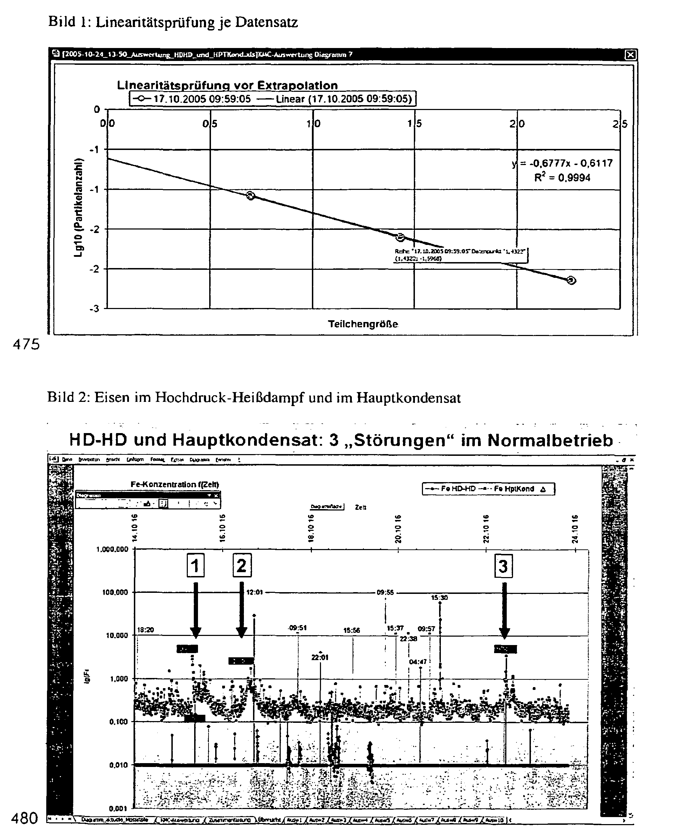

Das erfindungsgemäße Verfahren dient zur vollautomatischen Überwachung der Qualität der Wasser-Dampf-Kreisläufe von Kraftwerken zusätzlich zu den bisher eingesetzten Analysemessgeräten
a) mindestens ein nach Partikelgrößen klassifizierender Online-Partikelzähler eingesetzt, der
b) Anzahl und Größenklassen der im Messbereich liegenden Partikel hinreichend genau bestimmt, so dass der innerhalb eines bestimmten Partikelgrößenbereiches gefundene funktionale, oft in doppeltlogarithmischer Darstellung lineare Zusammenhang zwischen der Partikelgröße und der Partikelanzahl (Bild 1) sowohl eine Plausibilitätskontrolle des Messwertdatensatzes ermöglicht als auch eine korrekte Extrapolation für die im Messgut vorhandenen kleineren Partikel unterhalb der Größenklassen-Erfassungsgrenze des verwendeten Online-Partikelzählers erlaubt, um durch Mitberücksichtigung dieser Anteile mit ausreichender Genauigkeit den Gesamtgehalt an Partikeln in der Probe zu ermitteln und
c) aus diesem durch ein Berechnungs- und Näherungsfaktor, zum Beispiel durch das im Anwendungsbeispiel vorgestellte Verfahren, das Partikelgesamtvolumen und daraus
d) über einen Kalibrier- oder Näherungsfaktor, der sich aus der Dichte und dem Eisengehalt der ungelösten Eisenverbindungen ergibt, die Konzentration des ungelösten Eisens im Probenstrom mit ausreichender Genauigkeit auch in solchen Proben zuverlässig zu überwachen, in denen die Partikel- bzw. Eisenoxidkonzentration relativ gering und/oder die vorliegenden Partikel bzw. Eisenoxidteilchen relativ klein sind, wie es z. B. insbesondere im Speisewasser und Dampf bzw. Dampfkondensat von Kraftwerken überwiegend der Fall ist.

Die mit der Erfindung erzielten Vorteile bestehen insbesondere darin, dass die anlagenrelevanten Messwerte zur jeweils vorliegenden Kontamination des beprobten Wassers oder Dampfkondensates mit nur sehr kurzer zeitlicher Verzögerung zur Verfügung stehen. Außerdem wird der übliche Stichproben-Charakter der Bestimmung dieses Parameters durch üblicherweise manuelle Probenahme und anschließende Laboranalyse durch quasikontinuierliche Messreihen ersetzt, die zufällige Effekte weitgehend eliminiert. Außerdem lassen sich zufällige Einmal-Effekte zuverlässig von über längere, wenigstens mehrere aufeinander folgende Messungen sich wiederholt zeigenden Änderungen unterscheiden und somit die adäquaten Maßnahmen aus diesen Erkenntnissen treffen. Der hohe Automatisierungsgrad verringert zudem den individuellen Einfluss des Probenehmers ("menschlicher Faktor") und reduziert somit die daraus üblicherweise resultierende zusätzliche Variabilität der Messergebnisse.

Vorteilhaft bei der Umsetzung der Erfindung erweist es sich, wenn in Bezug auf den Patentanspruch:

### Zu a)

Als Partikelzähler vorzugsweise Geräte mit Sensoren eingesetzt werden, die einen dynamischen Messbereich von 1 - 200 µm abdecken.

### Zu b)

Zur Erhöhung der Qualität des berechneten Schätzwertes für den Gesamtvolumenanteil (als Summe aus dem direkt gemessenen und dem extrapolierten Anteil) aller Partikel im Messgut bzw. die Konzentration des ungelösten Eisens in der Probe eine ausreichende Genauigkeit der Erfassung der Partikelgrößenverteilung gegeben ist, weshalb der für das erfindungsgemäße Verfahren eingesetzte Online-Partikelzähler die Partikel in möglichst vielen, vorzugsweise aber mindestens 5 Größenklassen getrennt erfassen können sollte.

### Zu c)

Bei der Auswertung der kontinuierlichen Online-Überwachung der Partikelkonzentration an verschiedenen Stellen im Wasser-Dampf-Kreislauf von Kraftwerken wurde festgestellt, dass - allerdings abhängig von der Konzentration der Eisenoxide, die in der Regel in Form von Magnetitpartikeln vorliegen - weder die Vielzahl der kleinen Partikel < 1 µm noch die weit geringere Anzahl der stark ins Gewicht fallenden größeren Partikel > 25 µm vernachlässigt werden darf.

Überraschend wurde dabei gefunden, dass in den untersuchten Wasser-Dampf-Kreisläufen von Kraftwerken ein näherungsweise linearer Zusammenhang zwischen dem Logarithmus der Partikelgröße und dem Logarithmus der Partikelanzahl besteht, wenn die Auswertung **je Messdatensatz** auf den als zuverlässig erscheinenden Partikelgrößenbereich beschränkt wird. Dieser als zuverlässig erscheinende Partikelgrößenbereich hängt zum einen von der Spezifikation der Messzelle des Partikelzählers ab (Begrenzung der maximalen Gesamtpartikelanzahl, z.B. auf 10.000 Partikel/ml), zum anderen ist aus statistischen Gründen eine Mindestanzahl an Partikeln je Größenkanal für eine hinreichende Ergebnissicherheit erforderlich. Diese Mindestanzahl wird zum Beispiel im DVGW-Arbeitsblatt W 213-6 vom Juni 2005 mit 10 Partikel/ml angegeben, im Gegensatz hierzu kann bei ausreichend großem Probenvolumen je Datensatz aber auch schon eine Konzentration von nur 0,1 Partikel/ml als ausreichend angesehen werden, wie sich in der Praxis zeigte.

Zur Darstellung eines möglichst streng linearen Zusammenhanges zwischen Partikelanzahl und Partikelgrößenklasse kann es erforderlich sein, das Auswertungsverfahren an die Messeinrichtung anzupassen. Diese Anpassung kann z.B. darin bestehen, gemessene Partikel nahe der unteren Auflösungsgrenze des Meßgerätes bei der Auswertung nicht mit zu berücksichtigen, weil hier die systembedingten Unsicherheiten (Geräterauschen und Koinzidenzen) am größten sind.

Obwohl die tatsächliche Form der in der Probe gezählten Partikel stark variieren kann, ist die Bestimmung der tatsächlichen Form der einzelnen Partikel zur Erreichung des Zieles der Erfindung nicht erforderlich, da das Partikelvolumen in Wasser-Dampf-Kreisläufen von Kraftwerken in ausreichender Näherung aus der gemessenen Partikelgröße unter der Annahme einer einzigen geometrischen Form berechnet werden kann, wobei im hier betrachteten Anwendungsgebiet oft die Annahme würfelförmiger Partikel zu deutlich besseren Ergebnissen führt als die Annahme anderer Formen wie z.B. der Kugelform. Dies ist auch zu erwarten, denn zum Beispiel kristallisiert Magnetit und Wüstit im kubischen Kristallsystem (Hexaeder, Rhombendodekaeder, Oktaeder, Tetrakishexaeder, Trisoktaeder, Ikositetraeder, Hexakisoktaeder), häufig als Oktaeder, seltener als Rhombendodekaeder, Hämatit dagegen im trigonalen Kristallsystem (Basispinakoid, Prismen, Rhomboeder, Dipyramiden, Skalenoeder und Kombinationen). Im Wasser-Dampf-Kreislauf liegen die ungelösten Eisenoxidverbindungen aber auch in kolloidaler und amorpher Form als Eisenoxidhydrate vor, so dass zur Vereinfachung der Berechnung ein Formfaktor so festzulegen ist, dass sich eine möglichst gute Übereinstimmung der berechneten Eisenkonzentrationen im Wasser-Dampf-Kreislauf mit den tatsächlich gemessenen Eisenkonzentrationen im Konzentrationsbereich der vorgegebenen Grenz- und Richtwerte ergibt. Dies ist im vorgestellten Beispiel bei Annahme würfelförmiger Partikel der Fall.

### Zu d)

Zur näherungsweisen Umrechnung des über alle Korngrößenklassen aufsummierten Partikelvolumens in die gesuchte Konzentration des ungelösten Eisens kann als Näherungsfaktor, der sich aus der Dichte der als Partikel erfassten ungelösten Verbindungen und ihrem prozentualen Eisengehalt ergibt, ein Wert von maximal 2,97 beim Vorliegen von Hämatit bis maximal 4,57 beim Vorliegen von Wüstit angewendet werden, wenn die Partikel überwiegend aus den reinen Oxiden bestehen. Im Falle von reinem Magnetit wird ein Wert von maximal 3,77 verwendet, in anderen Fällen sind andere Umrechnungsfaktoren anzuwenden. Dabei kann der Näherungsfaktor alternativ auch aus der noch weniger stark schwankenden Dichte von 4,90 bis 5,88 und aus einer gelegentlich durchgeführten Analyse des Eisenanteiles im schonend getrockneten Membranfilterrückstand einer abfiltrierten Wasserprobe berechnet werden.

Die Herleitung der hier genannten Faktoren wird im Beispiel weiter ausgeführt.

In einer bevorzugten Ausführung des Verfahrens sollte die Probenentnahme und Partikelzählung kontinuierlich im Durchfluss erfolgen.

Die im normalen Anlagenbetrieb real auftretenden Schwankungen der Messergebnisse können durch eine Mittelung über mehrere in aufeinander folgenden Messzyklen ermittelte Messergebnisse geglättet werden, ohne dadurch die hohe Nachweisempfindlichkeit des Verfahrens als solche zu beeinträchtigen, womit verhindert wird, dass unmaßgebliche zufällige Ereignisse überbewertet werden, wobei eine selbst geringe tendenzielle Abweichung dennoch zuverlässig erkennbar bleibt.

Es erweist sich als zweckmäßig, die Probe zur Partikelgrößenanalyse saugend aus einem Überlaufgefäß zu entnehmen, das sowohl die Abscheidung störender Luftblasen ermöglicht als auch die ungestörte Entnahme einer Laborprobe für andere oder vergleichende Untersuchungen.

Um einen zuverlässigen automatischen Betrieb der Partikelzähler bzw. der Sensoren sicherzustellen, sollten Messsysteme bevorzugt werden, die sich selbst überwachen und die das optische System probewasserseitig bei Verschmutzung durch ungelöste Eisenverbindungen rechtzeitig automatisch selbst reinigen, wobei sich als Reinigungsflüssigkeit im Kraftwerksbereich verdünnte Salzsäure bewährt hat.

Zur Kostenminderung ist es zweckmäßig und oft ausreichend, Partikelzähler mit mehreren intern umschaltbaren Probeneingängen zu verwenden und - manuell oder programmgesteuert automatisch - vorübergehend jeweils nur den Probenwasserstrom oder zyklisch abwechselnd nur einen der Probenwasserströme durch den Sensor zu leiten, der aktuell von besonderem Interesse ist, weil entweder erhöhte Messwerte vorliegen oder ein Probenwasserstrom besonders intensiv überwacht werden soll, wie z.B. die Heißdampfkondensatprobe(n) vor und nach dem Anfahren der Dampfturbine.

Nach der Umschaltung des Probeneinganges kann es zweckmäßig sein, nicht nur eine kurze Probenspülzeit des Sensorsystems abzuwarten, sondern durch aufeinander folgende Messzyklen die Effektivität der Probenspülung zu überwachen, wobei für diese Überwachung drei aufeinander folgende Messzyklen als ausreichend angesehen werden können.

Es kann von Vorteil sein, mindestens 2 Sensoren je Kraftwerksblock einzusetzen, um den erfassten Partikelgrößen-Messbereich der Probenbeschaffenheit anzupassen, z.B. weil einzelne größere Partikel (z.B. um 100 µm) bei der Berechnung des Partikelvolumens wesentlich stärker ins Gewicht fallen als eine viel größere Anzahl kleiner Partikel um 1 µm, diese aber nur in stärker belasteten Kreislaufsystemen (Trommelkesselinhaltswasser) in einer auswertbaren Häufigkeit (vgl. auch DVGW Richtlinie 213-6, Juni 2005, zur Partikelmessung) auftreten.

Zur Überprüfung der Genauigkeit des Messverfahrens ist es von Vorteil, wenn am Ausgang der Messzelle des Partikelmessgerätes eine Membranfilter-Druckfiltration angeschlossen werden kann, wobei die geräteinterne Probenansaugpumpe gleichzeitig den Druck für die Membranfiltration bereitstellt. Im Kraftwerksbereich werden zur Abtrennung des ungelösten Eisens vereinbarungsgemäß Membranfilter mit einer Porenweite von 0,45 Mikrometern eingesetzt.

Die Kombination der Online-Bestimmung des ungelösten Eisens in der beschriebenen Form mit weiteren bereits vorhandenen bzw. dem Fachmann bekannten Einrichtungen zur Qualitätsüberwachung von Wasser-Dampf-Kreisläufen in Kraftwerken (Prozessleittechnik, automatisierte Probenahmesysteme, Messeinrichtungen zur temperaturkompensierten Bestimmung der elektrischen Leitfähigkeit vor und hinter stark saurem Probenahme-Kationenaustauscher und bei Erfordernis weiteren Messeinrichtungen zum Beispiel zur Bestimmung von pH-Wert, Redoxpotential, Natrium, Kieselsäure, Sauerstoff, Wasserstoff) ermöglicht die zuverlässige quasikontinuierliche automatische Überwachung und Dokumentation aller wesentlichen Qualitätsparameter.

### Ausführungsbeispiel

Eingesetzt wurde ein handelsüblicher Partikelzähler für Online-Messungen, der mit Hilfe einer internen kalibrierten Pumpe aus verschleißfester Keramik aus 2 ständig überlaufenden Probeflaschen von oben abwechselnd Probewasser aus der Trommelkesselabschlämmung des Niederdruck- und Hochdruckkessels, den Heißdampfkondensaten und dem Hauptkondensat einer Abhitzekesselanlage eines Gas- und Dampfturbinenkraftwerkes mit einer Durchflussrate von 25 ml/min. entnahm.

Der Sensor des bei den Praxiserprobungen eingesetzten Partikelmessgerätes war herstellerseitig nach ANSI/NFPA T2.9.6 R1.1990 mit Latex in Wasser im Bereich von 1 - 150 µm kalibriert. Die Messöffnung betrug laut Herstellerangabe 500 x 500 µm. Der Sensor konnte laut Spezifikation eine max. Konzentration von 24.000 Partikel/ml bei dem angegebenen Volumenstrom von 25 ml/min. erfassen.

Um ungestörte und zeitgleiche Laborprobenahmen zu ermöglichen, wurde an der ohnehin an jeder Probenahmestelle vorhandenen Laborprobenentnahmestelle ein Überlaufgefäß angeschlossen, aus dem auch der Partikelzähler das Messgut entnahm.

Die Dichte von Magnetit(Fe₃O₄)- und Hämatit(Fe₂O₃)-Kristallen beträgt ca. 4,9-5,2 bzw. 4,9-5,3 g/cm³, die Dichte von Wüstit(FeO) liegt mit 5,88 g/cm³ nur etwas darüber.

Der Eisengehalt von Fe₂O₃ beträgt ca. 56,0 Masse-%, in diesem Beispiel berechnet sich der Faktor zur Umrechnung des Partikelvolumens in die Eisenkonzentration zu 2,97, dies ist der maximal mögliche Faktor beim Vorliegen von reinem Hämatit. (1 g Fe₂O₃ = 0,560 g Fe; 1 cm³ Fe₂O₃ = 5,3 g Fe₂O₃ = ca. 2,97 g Fe).

Der Eisengehalt von Fe₃O₄ beträgt ca. 72,4 Masse-%, in diesem Beispiel berechnet sich der Faktor zur Umrechnung des Partikelvolumens in die Eisenkonzentration zu 3,77, dies ist der maximal mögliche Faktor beim Vorliegen von reinem Magnetit. (1 g Fe₃O₄ = 0,724 g Fe; 1 cm³ Fe₃O₄ = 5,2 g Fe₃O₄ = ca. 3,77 g Fe).

Der Eisengehalt von FeO beträgt ca. 77,7 Masse-%, in diesem Beispiel berechnet sich der Faktor zur Umrechnung des Partikelvolumens in die Eisenkonzentration zu 4,57, dies ist der maximal mögliche Faktor beim Vorliegen von reinem Wüstit. (1 g FeO = 0,777 g Fe; 1 cm³ FeO = 5,88 g FeO = ca. 4,57 g Fe).

Der tatsächlich vorliegende Eisengehalt wird aufgrund anderer Verunreinigungen in der Praxis eher etwas geringer sein, diese Abweichung beeinträchtigt aber nicht das Ziel der Erfindung, sondern stellt eine wünschenswerte Sicherheitsreserve dar, um vorgegebene Richt- und Grenzwerte für Eisen nicht zu überschreiten (konservative Abschätzung).

Messwerte über eine Periode von 10 Tagen sind beispielhaft in Bild 2 dargestellt, besonders hohe Online-Messwerte sind mit einer Zeitmarkierung versehen. Der anschließende Vergleich mit den in der Dokumentation zum Anlagenbetrieb festgehaltenen Ereignissen gab für die aufgetretenen erkennbar herausragenden Messwerte folgende Erklärungen:
- Punktuelle Änderungen der Eisenkonzentration als Folge von Durchflussänderungen an der Probenahmestelle bzw. störende Erschütterungen bei Handprobenahme am Morgen, resultierend aus einer Mobilisierung zuvor abgelagerter Eisenoxide. Probenahmefehler dieser Art sind in der Praxis bekannt.
- Vorübergehend andauernd erhöhte Eisenwerte in drei Fällen (mit Pfeil gekennzeichnet) während des ansonsten ungestörten Normalbetriebes für die Probenahmestellen Hochdruck-Heißdampf (HD-HD) und Hauptkondensat. Die nachträgliche Ursachenforschung ergab, dass genau mit diesen Zeitpunkten korrelierend durch das Prozessleitsystem plötzliche Rückgänge der Hochdruck-Heißdampfmenge zwischen 15% bis 20% registriert wurden.

## Patentansprüche

1. Verfahren zur automatischen Überwachung der Qualität von Wasser-Dampf-Kreisläufen in Kraftwerken, **gekennzeichnet durch** folgende Verfahrensschritte
a) Bestimmung von Anzahlen und Größenklassen von Partikeln mittels wenigstens eines Online-Partikelzählers an einer Stelle im Wasser-Dampf-Kreislauf,
b) Bestimmung des funktionellen Zusammenhangs zwischen Partikelgröße und Partikelanzahl und anschließende Überprüfung des Vorliegens eines linearen Zusammenhangs zwischen dem Logarithmus der Partikelgröße und dem Logarithmus der Partikelanzahl,
c) Berechnung des Partikelgesamtvolumens und
d) Extrapolation des ermittelten funktionellen Zusammenhangs zwischen Partikelgröße und Partikelanzahl, um im Wasser-Dampf-Kreislauf vorhandene kleinere Partikel, deren Größen unterhalb der Größenklassen-Erfassungsgrenze des verwendeten Online-Partikelzählers liegen, zu bestimmen, wobei diese bei der Berechnung des Partikelgesamtvolumens mitberücksichtigt werden,
e) Berechnung der Konzentration von ungelöstem Eisen aus dem Partikelgesamtvolumen über einen Kalibrier- oder Näherungsfaktor, der sich aus der Dichte und dem Eisengehalt der ungelösten Eisenverbindungen ergibt.

2. Verfahren nach Anspruch 1, **dadurch gekennzeichnet, dass** zur Online-Partikelgrößenbestimmung ein optischer Sensor verwendet wird.

3. Verfahren nach einem der Ansprüche 1 oder 2, **dadurch gekennzeichnet, dass** zur Umrechnung der gemessenen Partikelzahl in das Partikelgesamtvolumen Formfaktoren verwendet werden.

## Claims

1. Method for automatic monitoring of the quality of water-steam circuits in power stations, **characterised by** the following method steps:
a) determining numbers and size categories of particles by means of at least one on-line particle counter at a location in the water-steam circuit,
b) determining the functional correlation between particle size and particle number and subsequent checking for the presence of a linear correlation between the logarithm of the particle size and the logarithm of the particle number,
c) calculating the particle total volume and
d) extrapolating the determined functional correlation between particle size and particle number so as to ascertain smaller particles, the sizes of which lie below the size category detection limit of the on-line particle counter used, present in the water-steam circuit, wherein these are taken into consideration in the calculation of the particle total volume, and
e) calculating the concentration of undissolved iron from the particle total volume by way of a calibrating or approximation factor, which results from the density and the iron content of the undissolved iron compounds.

2. Method according to claim 1, **characterised in that** an optical sensor is used for the on-line particle size determination.

3. Method according to one of claims 1 and 2, **characterised in that** shape factors are used for conversion of the measured particle count into the particle total volume.

## Revendications

1. Procédé de surveillance automatique de la qualité de circuits eau-vapeur dans les centrales électriques, **caractérisé par** les étapes de procédé suivantes :
a) détermination des nombres et classes de taille de particules au moyen d'au moins un compteur de particules en ligne à un emplacement dans le circuit eau-vapeur,
b) détermination de la relation fonctionnelle entre taille de particules et nombre de particules puis vérification de l'existence d'une relation linéaire entre le logarithme de la taille de particules et le logarithme du nombre de particules,
c) calcul du volume total de particules et
d) extrapolation de la relation fonctionnelle déterminée entre taille de particules et nombre de particules pour déterminer les particules plus petites présentes dans le circuit eau-vapeur, dont les tailles se situent au-dessous de la limite de détection de classe de taille du compteur de particules en ligne utilisé, celles-ci étant prises en compte dans le calcul du volume total de particules,
e) calcul de la concentration de fer non dissous à partir du volume total de particules par le biais d'un facteur d'étalonnage ou d'approximation qui est obtenu à partir de la densité et de la teneur en fer des composés de fer non dissous.

2. Procédé selon la revendication 1, **caractérisé en ce qu'**un capteur optique est utilisé pour la détermination en ligne de la taille des particules.

3. Procédé selon une des revendications 1 ou 2, **caractérisé en ce que** des facteurs de forme sont utilisés pour la conversion du nombre de particules mesuré en volume total de particules.
